(19) Europäisches Patentamt
European Patent Office
Office européen des brevets

(11) EP 2 096 166 A1

# (12) EUROPEAN PATENT APPLICATION

(43) Date of publication:
**02.09.2009 Bulletin 2009/36**

(21) Application number: **09007120.0**

(22) Date of filing: **24.03.2006**

(51) Int Cl.:
***C12N 15/02*** *(2006.01)* ***C12N 5/06*** *(2006.01)*
***C12P 21/08*** *(2006.01)* ***G01N 33/566*** *(2006.01)*

(84) Designated Contracting States:
**CH DE GB LI**

(30) Priority: **31.03.2005 JP 2005103072**

(62) Document number(s) of the earlier application(s) in accordance with Art. 76 EPC:
**06729949.5 / 1 870 456**

(71) Applicants:
- **Osaka University**
  **Suita-shi**
  **Osaka 565-0871 (JP)**
- **TOTTORI UNIVERSITY**
  **Tottori-shi, Tottori 680-8550 (JP)**
- **Biomedics Inc.**
  **Bunkyo-ku, Tokyo 1120004 (JP)**

(72) Inventors:
- **Uchiyama, Susumu**
  **Suita-shi, Osaka 5650871 (JP)**
- **Fukui, Kiichi**
  **Suita-shi, Osaka 5650871 (JP)**
- **Suzuki, Yasuhiko**
  **Sapporo-shi, Hokkaido 001-0020 (JP)**
- **Yokoyama, Masami**
  **Tokyo 1120004 (JP)**
- **Usuda, Sadakazu**
  **Tokyo 1120004 (JP)**

(74) Representative: **Forrest, Graham Robert et al**
**Mewburn Ellis LLP**
**33 Gutter Lane**
**London**
**EC2V 8AS (GB)**

Remarks:
This application was filed on 28-05-2009 as a divisional application to the application mentioned under INID code 62.

## (54) Method for production of antibody directed against cell membrane surface antigen epitope and assaying method

(57) Disclosed is a method for determining the affinity of an antibody capable of binding to a cell membrane surface antigen for the antigen; and a method for assaying or screening an antibody capable of binding to a cell membrane surface antigen by utilizing the determination method. In the determination of the affinity of the antibody for the antigen, floating cells presenting the antigen on the surface of the cell membrane thereof are used and the B/F separation of the cells is made by centrifugation or on a filter through which the cells cannot pass.

EP 2 096 166 A1

SEQUENCE LISTING

```
<110>  OSAKA UNIVERSITY
       TOTTORI UNIVERSITY
       BioMedics Inc.

<120>  Methods for production of antibody directed against cell membrane surfac
antigen epitope and assaying method

<130>  GRF/FP6624910

<140>  Divisional application of 06729949.5
<141>  2006-03-24

<150>  JP 2005-103072
<151>  2005-03-31

<160>  2

<210>  1
<211>  35
<212>  DNA
<213>  Artificial sequence

<220>
<223>  primer

<400>  1
aatgcggccg ccaccatgac aacacccaga aattc                                 35

<210>  2
<211>  29
<212>  DNA
<213>  Artificial sequence

<220>
<223>  primer

<400>  2
gctctagatt aaggagagct gtcattttc                                        29


1
```

## Description

TECHNICAL FIELD

The present invention relates to a method for producing a monoclonal antibody against a cell membrane surface antigen (an extracellular domain of a cell membrane antigen). Moreover, the present invention relates to a method for assaying or screening an antibody that binds to the cell membrane surface antigen.

BACKGROUND ART

A soluble antigen such as a cytokine, a monokine, or a free antigen from a cell membrane, is generally prepared by gene recombination so that E. coli bacterium or the like serves as a host by gene recombination, and some antigen proteins each containing a sugar chain are produced by gene recombination so that an animal cell, such as CHO cell, derived from a rodent serves as a host. On the other hand, a molecule expressed on a cell membrane such as a receptor or an adhesive molecule has an insoluble transmembrane portion, and therefore, generally, the entire molecule is separated and purified from a culture cell or an extracellular domain of the molecule is prepared by gene recombination (Francois Baneyx (Editor), Protein Expression Technologies: Current Status and Future Trends, BIOS Scientific Publishers, April 1, 2004. ; Barry Steven Selinsky (Editor), Membrane Protein Protocols: Expression, Purification, and Characterization., Methods in Molecular Biology, V.228, Clifton, N.J., June 1, 2003).

However, a receptor or an adhesive molecule is not always easy to be prepared as an antigen thereof because of an aspect or an expression manner thereof. For example, a cell membrane antigen molecule is not a monomer but occasionally forms a complex, many cases that the molecule cooperates with another membrane molecule to exert the function are reported. For example, there are CD3/TCR, CD11a/CD18, CD49b/CD29, and so forth. Therefore, for producing a monoclonal antibody recognizing an epitope or a conformation of the antigen presented in a natural state, the method in which a molecule prepared by gene recombination with setting E. coli or a yeast to be a host is used as an antigen is not necessarily appropriate. Moreover, in such cell membrane antigens, as well as the transmembrane portion, the extracellular domain is occasionally insoluble or difficult to be solubilized. The conformation may be presented in the same manner as the natural state, only on a cell membrane surface of an animal cell. For example, it is thought that CD20, FcεRI, or MDR1 corresponds thereto.

It is known to be difficult to produce an antibody against a specific epitope of a cell membrane surface antigen. For example, it is known that the extracellular domain of CD20 antigen is insoluble and has an epitope getting involved in a signal induction of apoptosis, but it is supposed to be very difficult to obtain a high-affinity specific antibody recognizing the epitope according to many documents (Julie P.D. et al., Imminol 2002; 107:176-82; Maria J. Polyak and Julie P. Deans, Studies of Existing CD Molecules:93-95; and M. S. Cragg et al., Studies of Existing CD Molecules:95-97). Therefore, it is well-known that murine antibody 2B8, which is an origin of Rituxan (trademark), has been obtained by immunizing SB cell line, which is a human B cell, and 2H7 has been also obtained by using 6.16c1.3, which is a human B cell, and that the clones producing the antibodies have been extremely difficult to be obtained (Anderson KC et al., Blood 1984; 63:1424-33, Data Sheet: FITC labeled CD20, Medical & Biological Laboratories Co., Ltd.). For using these human cell lines as immunogens, occasionally, the expression amount of the desired antigen on a cell membrane surface is not sufficient, and also occasionally, sufficient immune response to the desired antigen cannot be obtained because the cell is of a different species from that of an animal to be immunized that is generally used (mouse or rat) and therefore the entire cell containing the cell membrane antigen has antigenicity.

DISCLOSURE OF THE INVENTION

Accordingly, an object of the present invention is to provide a method for producing a hybridoma producing a monoclonal antibody against a cell membrane surface antigen and a method for producing the monoclonal antibody against the extracellular membrane surface antigen by using the hybridoma. Moreover, another object of the present invention is to provide a method for measuring affinity of an antibody that binds to a cell membrane surface antigen and a method for assaying or screening the antibody that binds to the cell membrane surface antigen by utilizing the measuring method.

The present inventors has repeated trial and error on immunization methods for producing an antibody recognizing CD20 antigen, and therefore, found that a monoclonal antibody which competitively reacts with the antibody c2B8, which is known to be useful as a medicine, can be obtained by a method of immunization with a specific combination. It has been clarified that according to the immunization method, the percentage of clones producing the antibody against the desired cell membrane surface antigen out of the clones to be obtained has significantly increased. Moreover, it has been found that according to a simple assay method by using a secondary antibody labeled with a fluorescent dye, binding affinity between the cell membrane surface antigen and the antibody against the antigen can be measured at a high sensitivity. The present invention has been accomplished based on the findings.

The present invention relates to the following.

(1) A method for producing a hybridoma producing a monoclonal antibody recognizing an epitope in an extracellular domain of a cell membrane antigen, which comprises immunizing an animal to be immunized at a plurality of times, wherein at least one of the immunizations is an immunization by using a cell strain that expresses the antigen as a sensitizing antigen and that is derived from an animal belonging to a different order from that of the animal to be immunized, and at least one of the immunizations is an immunization by using a cell strain that is made to express the antigen as the sensitizing antigen on a cell membrane surface by gene recombination and that is derived from an animal belonging to a same order as the animal to be immunized.
(2) The method according to (1), wherein each of the plurality of immunizations is either an immunization by using a cell strain as a sensitizing antigen that expresses the antigen and that is derived from an animal belonging to a different order from that of the animal to be immunized, or an immunization by using a cell strain as a sensitizing antigen that is made to express the antigen on a cell membrane surface by gene recombination and that is derived from an animal belonging to a same order as the animal to be immunized.
(3) The method according to (2), which comprises performing initial immunization, additional immunization(s) and final immunization; wherein at least one immunization of the initial immunization and the additional immunization(s) is the one immunization which is either an immunization by using a cell strain as a sensitizing antigen that expresses the antigen and that is derived from an animal belonging to a different order from that of the animal to be immunized, or an immunization by using a cell strain as a sensitizing antigen that is made to express the antigen on a cell membrane surface by gene recombination and that is derived from an animal belonging to a same order as the animal to be immunized; and the final immunization is the other immunization.
(4) The method according to any one of (1) to (3), wherein the cell membrane antigen is a molecule expressed in a normal animal cell or in an animal cell strain made to be a cell line.
(5) The method according to (4), wherein the normal animal cell or the animal cell strain made to be a cell line expressing the cell membrane antigen is a lymphocyte.
(6) The method according to any one of (1) to (4), wherein the cell membrane antigen is a transmembrane molecule.
(7) The method according to any one of (1) to (5), wherein an extracellular domain presenting the epitope of the cell membrane antigen is an antigen that is insoluble or difficult to be solubilized.
(8) The method according to any one of (1) to (7), wherein the animal to be immunized is an animal belonging to the order rodentia, the cell strain derived from an animal belonging to a same order as the animal to be immunized is CHO cell, NSO cell, or SP2/o cell.
(9) A method for producing the monoclonal antibody, wherein the hybridoma produced by the method according to any one of (1) to (8) is used.
(10) A method for measuring binding affinity between an antibody to be measured and a cell membrane surface antigen, which comprises (a) binding the antibody to be measured to a floating cell presenting the antigen; (b) separating the antibody to be measured bound to the cell membrane surface antigen of the floating cell from the free antibody to be measured; (c) binding a labeled substance that binds to the antibody to be measured at a site different from the antigen recognizing site thereof, to the antibody to be measured bound to the cell membrane surface antigen of the floating cell; (d) separating the substance binding to the antibody to be measured binding to the cell membrane surface antigen of the floating cell from the free substance; and (e) detecting the label of the substance; and wherein the separations of (b) and (d) are each performed by centrifugation or through a filter through which a cell cannot pass.
(11) The method according to (10), wherein the separations of (b) and (d) are performed by centrifugation.
(12) The method according to (10), wherein the separations of (b) and (d) are performed through the filter through which a cell cannot pass.
(13) The method according to any one of (10) to (12), wherein the floating cell is a normal animal cell, an animal cell strain made to be a cell line, or an animal cell strain modified genetically.
(14) A method for assaying the monoclonal antibody that binds to the cell membrane antigen by utilizing the method according to any one of (10) to (13).
(15) A method for screening the monoclonal antibody that binds to the cell membrane antigen by utilizing the method according to any one of (10) to (13).

## EFFECT OF THE INVENTION

According to a method for producing a hybridoma of the present invention, a hybridoma producing a monoclonal antibody against a cell membrane surface antigen can be efficiently produced. In particular, a hybridoma producing a monoclonal antibody against a cell membrane surface antigen that is difficult to present a natural conformation by separation and purification or only by mere gene recombination, against which it has been supposed to be difficult to produce an antibody, can also be produced. Moreover, by producing such a hybridoma, a large amount of monoclonal antibodies against cell membrane surface antigens.

According to the measuring method of the present invention, affinity of an antibody against a cell membrane surface antigen can be measured at a high sensitivity, and also, a method for assaying or screening the antibody by utilizing the measuring method is provided. The measuring method of the present invention is particularly suitable for measurement of affinity of a monoclonal antibody binding a natural epitope of a cell membrane surface antigen or an extracellular domain thereof, of which binding affinity is difficult to be measured by a general method.

BRIEF DESCRIPTION OF THE DRAWINGS

Fig. 1 shows the structure of pNOW, which is a vector for protein expression.
Fig. 2 shows a measurement result by an image analyzer (photograph of grey-level images).
Fig. 3 shows a saturated curve. (X Axis: Amount of Added Antibody (ng), Y Axis: Fluorescence Intensity)
Fig. 4 shows a result of Scatchard analysis. (X Axis: Bound (nM), Y Axis: Bound/ Free)

| | |
|---|---|
| Pcmv: | CMV promotor |
| Pabgh: | polyA addition signal of the growth hormone gene |
| Psvd: | modified SV40 promoter |
| DHFR: | mouse dihydrofolate reductase cDNA |
| Pasv: | polyA addition signal of SV40 gene |
| PBR322ori: | replication origin in *Escherichia coli* |
| Ampr: | selective marker in *Escherichia coli* (ampicillin resistance) |
| Neor: | selective marker in mammalian cell (G418 resistance) |

BEST MODE FOR CARRYING OUT THE INVENTION

<1> Method for Producing Hybridoma and Method for Producing Monoclonal Antibody according to the Present Invention

The method for producing a hybridoma according to the present invention is characterized by a method for producing a hybridoma producing a monoclonal antibody recognizing an epitope in an extracellular domain of a cell membrane antigen, including immunizing an animal to be immunized at a plurality of times, in which at least one of the immunizations is an immunization by using a cell strain as a sensitizing antigen that expresses the antigen and that is derived from an animal belonging to a different order from that of the animal to be immunized, and at least one of the immunizations is an immunization by using a cell strain as the sensitizing antigen that is made to express the antigen on a cell membrane surface by gene recombination and that is derived from an animal belonging to a same order as the animal to be immunized.

The method for producing a hybridoma according to the present invention may be the same as a method for producing a hybridoma producing a general monoclonal antibody except that the immunization of the animal to be immunized is performed in the above specific manner. A hybridoma producing a monoclonal antibody is generally produced by (1) immunization of an animal to be immunized, (2) preparation of a lymphocyte, (3) preparation of a parent cell, (4) cell fusion of the lymphocyte and the parent cell, and (5) screening and cloning. Particulars about the respective steps thereof are described, for example, in Monokuronal kotai, Seikagaku Jikkenho (Monoclonal Antibody, Biochemical Experiment Method), edited by Ailsa M. Campbell, and translated by Toshiaki OSAWA, Tokyo Kagaku Dojin (1989).

The animal to be immunized is not particularly limited, but is preferably an animal to be generally used for producing an antibody. Such animals include rodents, more specifically, mouse, rat, and hamster.

The immunization of the animal to be immunized is performed by performing injection (immunization) of a sensitizing antigen at a plurality of times. The respective immunization conditions such as number of plural immunizations, interval of the immunization, and injection amount of the sensitizing antigen, may be performed by general methods except that the immunization of the animal to be immunized is performed in the above specific manner. In general, when the immunization is performed at three times or more, the first immunization is referred to as first immunization, and the final immunization is referred to as final immunization (boost), and immunization after the first immunization and before the last immunization is referred to as additional immunization, and conditions suitable therefor are selected.

In the method for producing a hybridoma according to the present invention, it is sufficient that at least one of the immunizations is an immunization by using a cell strain as a sensitizing antigen that expresses the antigen and that is derived from an animal belonging to a different order from that of the animal to be immunized, and that at least one of the immunizations is an immunization by using a cell strain that is made to express the antigen as the sensitizing antigen on a cell membrane surface by gene recombination and that is derived from an animal belonging to a same order as the animal to be immunized. Therefore, the order of the immunizations is not limited.

In the method for producing a hybridoma according to the present invention, it is preferable that each of the plural immunizations is either an immunization by using a cell strain as the sensitizing antigen that expresses the antigen and that is derived from an animal belonging to a different order from that of the animal to be immunized, or an immunization by using a cell strain as the sensitizing antigen that is made to express the antigen on a cell membrane surface by gene recombination and that is derived from an animal belonging to a same order as the animal to be immunized. Furthermore, it is preferable that the method including: performing initial immunization, additional immunization(s), and final immunization; in which at least one immunization of the initial immunization and the additional immunization(s) is the one immunization which is either an immunization by using a cell strain as a sensitizing antigen that expresses the antigen and that is derived from an animal belonging to a different order from that of the animal to be immunized, or an immunization by using a cell strain as a sensitizing antigen that is made to express the antigen on a cell membrane surface by gene recombination and that is derived from an animal belonging to a same order as the animal to be immunized; and the final immunization is the other immunization.

The cell membrane antigen is not particularly limited and includes CD20, TNFR (tumor necrosis factor receptor), and TGF-β (tumor growth factor) receptor. It is preferable that the cell membrane antigen is a molecule expressed in a normal animal cell or in an animal cell strain made to be a cell line. The normal animal cell or the animal cell strain made to be a cell line expressing the cell membrane antigen includes a lymphocyte.

By the method for producing a hybridoma according to the present invention, a hybridoma producing a monoclonal antibody against such an antigen even when the cell membrane antigen is a transmembrane molecule or even when a extracellular domain presenting an epitope of the cell membrane antigen is an antigen that is insoluble or difficult to be solubilized, in which it has been difficult to produce a monoclonal antibody against the cell membrane antibody by a conventional method.

The cell strain derived from an animal belonging to a different order from that of the animal to be immunized is not particularly limited as long as expressing the cell membrane antigen to be desired, and selected according to the animal to be immunized to be used. For example, when the animal to be immunized is a rodent, a cell strain derived from human can be used. For example, when the cell membrane antigen is CD20, the cell strain derived from human includes SB cell and Raji cell.

It is preferable that the expression amount of the cell membrane antigen in the cell strain derived from an animal belonging to a different order from that of the animal to be immunized is larger. For example, it is desirable that when the expression amount is detected by staining with CBB after SDS electrophoresis or by using an antibody against the antigen by western blotting method, there are at least several micrograms of the antigen per $10^7$ cells.

The cell strain that is made to express the antigen as the sensitizing antigen on a cell membrane surface by gene recombination and that is derived from an animal belonging to a same order as the animal to be immunized is not particularly limited as long as expressing the cell membrane antigen to be desired, and selected according to the animal to be immunized to be used. For example, when the animal to be immunized is a rodent, the cell strain includes CHO cell, NSO cell, and SP2/o cell.

It is preferable that the expression density of the cell membrane antigen in the cell membrane surface of the cell strain derived from an animal belonging to a same order as the animal to be immunized. For example, when the expression cell is stained with a monoclonal antibody labeled with FITC, it is preferable that the fluorescent intensity which is five or more times stronger than that of the cell inherently having the antigen is detected.

For example, recombinant CHO cell expressing CD20 (CD20/CHO) can present a predominantly larger amount of CD20 molecules on the cell surface than that of a natural B cell strain, and is effective as an immunogen. It has been revealed that the CD20/CHO antigen transfected with pNOW having the entire sequence of CD20 is presented at an extremely high density although depending on performance of the recombinant protein expression vector. This has been proved by a binding reaction test by using an anti-CD20 monoclonal antibody labeled with FITC that is commercially available (DAKO, catalog number F0799).

Moreover, the present invention provides a method for producing the monoclonal antibody, in which the hybridoma produced by the method according to the present invention is used.

The method for producing the monoclonal antibody by using the hybridoma may be the same as a general method for producing a monoclonal antibody except that the hybridoma produced by the method for producing a hybridoma according to the present invention. In the case of a large amount of monoclonal antibody, a method by cell culture and a method by production as a mouse ascites fluid can be exemplified.

<2> Method for Measuring Affinity according to the Present Invention

The method for measuring affinity according to the present invention is characterized by a method for measuring binding affinity between an antibody to be measured and a cell membrane surface antigen, including: (a) binding the antibody to be measured to a floating cell presenting the antigen; (b) separating the antibody to be measured bound to the cell membrane surface antigen of the floating cell from the free antibody to be measured; (c) binding a labeled substance that binds to the antibody to be measured at a site different from the antigen recognizing site thereof, to the antibody to be measured bound to the cell membrane surface antigen of the floating cell; (d) separating the substance binding to the antibody to be measured bound to the cell membrane surface antigen of the floating cell from the free substance; and (e) detecting the label of the substance; and in which the separations of (b) and (d) are each performed by centrifugation or through a filter through which a cell cannot pass.

The method for measuring affinity according to the present invention may be the same as a general method for measuring affinity between a antigen and an antibody except that a floating cell presenting an antigen on the cell membrane surface and that a labeled substance being capable of binding to the antibody to be measured at a site of the antibody to be measured different from a site thereof recognizing the antigen and that the separations of bound and free (B/F separation) are each performed by centrifugation or through a filter through which a cell cannot pass. However, it is preferable that the amount of the floating cell is set to approximately $5 \times 10^5$ cells per one tube in this measurement system. Alternatively, the measurement can be also performed by using a secondary antibody without directly labeling the antibody with RI.

For example, the substance being capable of binding to the antibody to be measured at a site of the antibody to be measured different from a site thereof recognizing the antigen includes an antibody against the antibody to be measured (for example, antibody against Fc site), Protein A, and Protein G. The label includes a fluorescent substance and a magnetic bead.

It is preferable that in the method for measuring affinity of the present invention, the separation method is the same.

The cell to be used is not particularly limited as long as being a floating cell presenting the antigen to be desired on the cell membrane surface. However, it is preferable that the cell is a normal animal cell, an animal cell strain made to be a cell line, or an animal cell strain modified genetically. When the antigen is CD20, such a cell includes Raji Cell.

The specific procedure is exemplified as follows. (1) The floating cell and a mouse monoclonal antibody (primary antibody) are reacted, and then, (2) the free primary antibody is removed by rinse and centrifugation, and then, (3) anti-mouse secondary antibody labeled with fluorescence is reacted therewith, and (4) the free secondary antibody is removed by rinse and centrifugation, and then (5) the fluorescent amount of the secondary antibody bound to the cell through the primary antibody is measured by a fluorescent image analyzer. Furthermore, (6) a standard curve on the used secondary antibody labeled with fluorescence is drawn by measuring the concentration and the fluorescent amount, and the measured value is converted into, and thereby, the amount of the bound secondary antibody is obtained, and the amount of the free antibody is obtained by subtracting the bound antibody amount from the added antibody amount.

The more specific procedure includes a method composed of the steps as described later.

1) A cell or a cell line expressing a large amount of the antibody on the cell membrane is selected and cultured until sufficient number of the cells is obtained.
2) The cells are collected by centrifugation and rinsed.
3) The cells are suspended in a solution containing the monoclonal antibody to be detected or a positive control antibody.
4) After shaken and reacted for a predetermined time, the cells are collected by centrifugation and quickly rinsed.
5) The collected cells are suspended in a solution containing the secondary antibody labeled with fluorescent.
6) After shaken and reacted for a predetermined time, the cells are collected by centrifugation and rinsed.
7) The cells are suspended again and transferred to wells in a 96-well plate, and the fluorescent intensity is detected by an image analyzer.
8) After the detection, the amount of the binding antibody and the amount of the free antibody are digitalized and the dissociation constant (Kd value) is obtained from the Scatchard plot.

In the present method, the cells are used without being fixed with formaldehyde and such, and therefore, the affinity between the cell surface membrane surface antigen to be desired with maintaining the conformation on a cell surface and the monoclonal antibody can be measured.

By utilizing the method for measuring affinity according to the present invention, a monoclonal antibody that can bind to the cell membrane surface antigen can be assayed or a monoclonal antibody binding to the cell membrane surface antigen can be screened. The assaying and screening methods may be the same as a general method for assaying a monoclonal antibody binding the a cell membrane surface antibody and a general method for screening a monoclonal antibody binding to a cell membrane surface antigen.

The reason why the effect of the present invention can be obtained can be thought as follows despite being restricted by the following explanation.

It is thought that by combining a cell of a different species from that of the animal to be immunized (a cell strain that expresses the antigen and that is derived from an animal belonging to a different order from that of the animal to be immunized) and a cell of a same species or a related species (a cell strain that is made to express the antigen on a cell membrane surface by gene recombination and that is derived from an animal belonging to a same order as the animal to be immunized) as the sensitizing antigens, immunogenicity of only the molecule to be desired is enhanced and therefore the proportion of the hybridoma producing a specific monoclonal antibody increases.

As seen from the knowledge that the antibody c2B8 is available as a medicine, it is thought that the antibody c2B8 recognizes the epitope in a natural state. And, the obtained large number of clones each producing a monoclonal antibody competing the antibody in the binding reaction with DC20 suggests that the molecular to be desired in the method of the present invention is in a natural state. This advantage is effective when the conformation of the epitope on the molecule to be desired cannot be realized in a general antigen preparation method. The general antigen preparation method is a method for solving cells expressing the antigen molecule and for separating and purifying the antigen. It is thought that the conformation of the antigen in a natural state can be realized by expressing the antigen on the cell surface. However, it is difficult to obtain a monoclonal antibody to be desired only by using a cell expressing such an antigen on the cell surface as an antigen. It is thought that by using such a specific combined immunization as described above, a monoclonal antibody to be desired in a practical proportion in such a case becomes possible to be obtained.

As the reason why the sensitivity is enhanced by using a floating cell in the affinity measurement, it is thought that the cell amount is limited because of the area of the solid phase surface upon stabilization, and on the other hand, the cell amount can be enlarged. Moreover, it is also one of the reasons that the antibody can bind to the antigens on the entire surface in a cell in a floating state, and by contrast, the cell surface area to which an antibody can bind is reduced by stabilization. In addition, in the case of cross-linking to make the cell a solid phase, there is possibility that the epitope occasionally disappears through the solid phase procedure or that the conformation thereof is changed, and there is possibility that the affinity against the antigen in a natural state cannot be measured or that the affinity is evaluated to be low, but there is not such possibility in the method for measuring affinity according to the present invention.

## Example 1

Production of a monoclonal antibody against CD20 molecule having an insoluble membrane surface antigen and quantitative measurement of the binding affinity of the obtained antibody will be described with reference to Example.

### <1> Production of Monoclonal Antibody

#### (1) Preparation of Immunization Antigen for Sensitizing Mouse

First, SB cell and Raji cell, which were presentative B cell lines expressing CD20, were cultured in vitro. Next, from Multiple Choice cDNA human spleen, Origene Technologies, Inc. 6 Taft Court Suite 100 Rockville, MD 20850, DNA encoding the entire molecule of CD20 was cloned by using a specific primer hCD20-S-GK-Not aatgcggccgccaccatgacaacacccagaaattc (Sequence No. 1) and hCD20-E-Xba gctctagattaaggagagctgtcattttc (Sequence No. 2) and incorporated into pNOW (Patent No. 3582965, Fig. 1), which was a high-expression vector for an animal cell, and the constructed vector was transfected into CHO cell. Thereby, a recombinant CHO cell (CD20/CHO cell) highly expressing CD20 molecule on the cell surface was established. In addition, the cell whose fluorescent intensity is five or more times stronger than that of SB cell in the case of staining with CD20 monoclonal antibody labeled with FITC was set to the highly-expressing cell.

#### (2) Preparation of Immunogen

SB cell or Raji cell was cultured by using RPMI1640 medium to which 10% FCS was added.

CD20/CHO cell was cultured by using CHO-S-SFM II medium (GIBCO, Cat. No. 12052-098) to which 800 pg/ml of G418 was added. After centrifugally separating the media (1100 rpm, 5 min), Dulbecco's PBS(-) was added to the cells and the cells were suspended, and centrifugally separated again. The rinse operation was repeated again, and, a normal saline solution was added to the cells and thereby suspensions ($1\times10^7$ to $3\times10^7$ cells per ml) were prepared, and used for immunization.

(3) Immunization

Both of the preparations of the immunogens were administered into an abdominal cavity of Balb/c-based female mouse of 7- to 11-week age. The combination of the used immunogens was shown in Table 1. The same cells of any one of SB cell, Raji cell, and CD20/CHO cell were repeatedly administered at 2 to 3 times with an interval of some days, and then, a different cell thereof was administered in the final immunization. The number of the administered cells was $1\times10^7$ to $3\times10^7$ cells per mouse in each of the cases thereof.

(4) Cell Fusion

After 3 days from the final immunization, spleen cells were prepared from two mice, and fusion reaction with mouse myeloma (NS-1) was performed under existence of PEG-1500. The method was according to Oi,V.T. and L.A. Herzenberg, 1980, in:Selected Methods in Cellular Immunology, eds. B. Mishell and S.M. Shiigi(Freeman and Co.San Francisco, CA) p.351.

(5) Primary and Secondary Screenings

Cell ELISA was performed by using 96-well plate to which CD20/CHO cells or CHO cells (parent strain) were attached, and wells producing an antibody reacting specifically with CD20 were selected. Furthermore, the same 96-well plate to which CD20/CHO cells were attached, and competitive reaction with an antibody c2B8 (human and mouse chimeric antibody produced from anti-CD20 mouse monoclonal antibody 2B8), which was known to be a medicine, was performed and the antibodies (wells) reacting with an analogous site to the epitope of c2B8.

(6) Cell ELISA

CD20/CHO cells or CHO cells (parent cell) attached to the 96-well plate coated with POLY-L-Lysine (ASAHI TECHNOGLASS CORPORATION, Cat. No. 11-023-018) were used for Cell ELISA. 150 μl of blocking solution (PBS solution of 0.2%-Gelatine, 0.5%-BSA) was put into each of the wells, and the respective wells were standing at 37°C for one hour. The plate is rinsed at 5 times by using an aqueous solution of 150 mM-NaCl, 0.05%-Tween20, and then, 100 μl of the samples (diluted solution of culture supernatant was put into each of the wells. After rinse, 100 μl of a diluted solution of labeled antibody [anti-mouse HRP-labeled IgG (H+L) rabbit antibody (Jackson Lab. Code No. 315-035-003), or anti-mouse HRP-labeled IgG (Fcγ) rabbit antibody (Jackson Lab. Code No. 315-035-008)] was put into each of the wells, and the secondary reaction was performed at 37°C for one hour. For preparation of the primary and secondary reaction solutions, the same solution as the blocking solution. After rinse, 100 μl of coloring solution (OPD) was put into each of the wells, and after 30 minutes, the reaction was stopped by adding 50 μl of 4N-H2SO4 thereto, and A492 was measured.

(7) Competitive Reaction in Cell ELISA

A mixed solution of the sample (diluted solution of the culture supernatant) and the chimeric antibody (10 to 40 ng/ml) was prepared.
In the same manner as Cell ELISA, after the blocking reaction, 100 μl of the mixed solution was put into each of the wells and the primary reaction was performed at 37°C for one hour. After rinse, 100 μl of a diluted solution of labeled antibody [anti-human HRP-labeled IgG (H+L) rabbit antibody (Jackson Lab. Code No. 309-035-082)] was put into each of the wells and the secondary reaction was performed at 37°C for one hour. After rinse, 100 μl of coloring solution (OPD) was put into each of the wells, and after 30 minutes, the reaction was stopped by adding 50 μl of 4N-H2SO4 thereto, and A492 was measured.
The labeled antibody was reacted with only the chimeric antibody, and therefore, when the antibody in the sample added in the primary reaction competed with the chimeric antibody, it was recognized that the measured value was lowered.

(8) Cloning

Cloning was performed by limiting dilution. The cells were dispersed on a 96-well plate and cultured, and then, culture supernatants in the wells each containing one colony were subjected to Cell ELISA and the clones each producing a specific antibody were selected.

(9) Preparation of Purified Antibody

The clone producing a specific antibody was cultured in RPMI1640 to which 10% FCS was added, and when the cell density became approximately 5×106 cells/ml, the medium was exchanged to serum-free medium ASF-104N (Ajinomoto Co. Inc.). After 2 to 4 days thereof, the culture solution was centrifugally separated and the culture supernatant was collected and then purified by using Protein G column, and the eluted monoclonal antibody solution was dialyzed with respect to 150 mM-NaCl. Filter sterilization was performed with 0.2 μm filter, and the antibody was set to test antibody (anti-human CD20 mouse monoclonal antibody).

<Results>

The results of the immunization method, the screenings of the hybridoma obtained by the immunization method, and the competitive reactions with c2B8, are shown in Table 1.

Table 1

| Cell fusion series | Immunization method | | | Primary and secondary screenings Specificity of CHO cell to CD20/CD20 | | |
|---|---|---|---|---|---|---|
| | Numbers of initial and additional immunization (s) | Final immunization | Number of immunized mice | Number of wells A | Number of wells B | Number of measured wells |
| 1K18 | SB cell, 3 times | Raji cell | 2 | 7 | 2 | 576 |
| 1K20 | Raji cell, 3 times | SB cell | 2 | 7 | 0 | 576 |
| 1K14 | SB cell, 2 times | CD20/CHO cell | 1 | 20 | 9 | 576 |
| | SB cell, 3 times | CD20/CHO cell | 1 | | | |
| 1K17 | CD20/CHO cell, 2 times | Raji cell | 1 | 21 | >10 | 576 |
| | CD20/CHO cell, 3 times | Raji cell | 1 | | | |

The Number of Selected Well-A: The number of wells producing an antibody reacting with CD20/CHO cell and not reacting with CHO cell

The Number of Selected Wells-B: The number of wells producing the antibody whose competitive reaction with control antibody (C2B8) can be recognized, out of Selected Well Number-A

As shown in Table 1, it was found that when both of the immunization by the cell derived from human (SB cell or Raji cell) and the immunization by CHO cell (CD20/CHO cell) that is made to express CD20 by gene recombination and that is derived from an animal belonging to a same order as mouse, a larger amount of the cells each producing a specific antibody against CD20 can be obtained by the cell fusion than that of the case of performing only immunization by the cell derived from human. Moreover, among the selected clones, a large number of clones each producing an antibody whose competitive reaction with the control antibody c2B8 can be recognized.

<2> Measurement of Binding Affinity

Floating cell Raji, which expressed an antigen to be desired and which was derived from human B cell line, was used, and floating cell Jurkat, which was derived from human T cell line, was used as the cell that did not express CD20 antigen. Both of the cells were cultured at 37°C in $5\%CO_2$ incubator (SANYO MCO-175M) on media in which 10% fetal bovine serum FCS (BIOLOGICAL IND. Cat.No.04-001-1A, Lot 815242, preliminarily heated at 56°C for 30 minutes for setting the complement component not to function) to RPMI1640 (Nakarai Co., Ltd., Cat.No. 30264-85, Lot L4K2844), and maintained by passage at twice a week.

The measurement of the cell number was performed by using Burker-Turk blood cell counter (ERMA, Inc. Cat.No.03-303-1).

The confluent-cell culture medium in third or fourth day after passage was centrifuged at room temperature at 3,000 rpm for 3 minutes by Low Temp Multi-Tube Centrifuge LX-120 (TOMY Co. Ltd.) and the supernatant was removed and the cells were collected. The rotational frequency and time were the conditions in which the cell number was not changed when the centrifugal separation and the removal of the supernatant were repeated. For removing the residual medium and FCS on the surface of the cell (rinse), the collected cells were suspended in Dulbecco's Phosphate Buffered Saline(-) without Ca and Mg [PBS(-), (NaCl:Wako, Cat.No.191-01665, $Na_2HPO_4$:Wako, Cat.No.197-02865, Lot ASF2635, KC1:Wako, Cat.No.163-0334T, Lot CEQ7122, $KH_2PO_4$:Wako, Cat.No.169-0425, Lot ELG7616)], and then, centrifuged at 3,000 rpm for 3 minutes, and the supernatant was removed, and the operations were performed twice. The cells rinsed were suspended in 1% BSA (Wako Cat No.013-07492 Lot PKH3483)-PBS solution, and the cell density was adjusted to $5\times10^6$ cells/ml.

As the primary antibody, 15, 30, 50, 75, 100, 125, 150, and 200 ng (1.5-5 μl) of test antibodies or positive control antibody (2B8) were each dispensed in 1.5 ml tube (BM Equipment Co., Ltd., BM-ring lock tube Cat.No.BM-15), and therewith, four tubes in which the antibody was not put were prepared. Moreover, three samples were prepared with respect to each of the test antibodies. 100 μl ($5\times10^5$ cells) of the suspension suspended by 1% BSA (Wako Cat No.013-07492 Lot PKH3483)-PBS solution was put in each of the tubes and mixed, and shaken and reacted at room temperature for one hour.

After the reaction, centrifugal separation was performed at room temperature at 3,000 rpm for 3 minutes by Low Temperature High Speed Refrigerative Centrifuge MX-100 (TOMY), and the cells were collected, and then, for removing unreacted primary antibody left on the surface of the cells, the cells were suspended in 200 μl of PBS and centrifuged at 3,000 rpm for 3 minutes and the supernatant was removed, and the operations were performed twice.

Next, for detecting the primary antibody binding to the cell, FITC-labeled anti-mouse IgG (H&L) secondary antibody [GOAT Anti-mouse IgG(H&L) Fluorescein conjugated, affinity purified Secondary antibody, Chemicon, Cat.No.AP124F, Lot 24021014] whose amount was excess (500 ng) with respect to the primary antibody binding the cell was added to the above cells and suspended, and shaken and reacted for one hour under dark condition and at room temperature. After the reaction, the cells were centrifuged at 3,000 rpm for three minutes, and collected, and then, for removing the unreacted FITC-labeled anti-mouse IgG (H&L) antibody left on the surface of the cells, the cells were suspended in 200 μl of PBS and centrifuged at 3,000 rpm for 3 minutes, and the supernatant was removed, and the operations were performed twice.

The cells thus obtained as described above were suspended in 100 μl PBS, and transferred to 96-well flat-bottom plate (SUMITOMO BAKELITE Co., Ltd., ELISA PLATE Cat.No.8496F). The fluorescent amount of the secondary antibody was measured by using Typhoon9210 image analyzer (Amersham Bioscience) under the detection condition of Fluorescense mode, 600v, 526SP/green (532nm), Focus bottom face +3 mm. In this case, the PBS solutions of 100 μl to which 0, 12.5, 25, 50, 75, 100, 125, and 150 ng of FITC-labeled secondary antibody were added were used as the control for drawing the standard curve.

After the detection, the image was digitized by using the image analysis software Image Quant (Amersham Bioscience), and then the analysis by Excel (Microsoft) was performed. In this case, as the background value derived from the plate and the PBS solution and the FITC-labeled secondary antibody binding nonspecifically to the cells, the measurement values of the case in which only the cells and the FITC-labeled secondary antibody were reacted were obtained, and the average of the four points was subtracted from each of the values of the fluorescent intensities of the samples. Thereby, the fluorescent amount of the FITC-labeled secondary antibody binding the cells was obtained. Furthermore, the standard curve was drawn by measuring the fluorescent amount in each of the concentrations of the FITC-labeled secondary antibody used as control, and thereby the amount (number of moles or weight) of the secondary antibody binding to the cells was obtained. Under the assumption that the primary antibody and the FITC-labeled secondary antibody ware reacted at a proportion of 1:2 respectively, the amount of the binding primary antibody was obtained. Moreover, the free primary antibody amount was obtained by subtracting the binding amount from the added amount. When the antibody concentration was converted into molar concentration, the molecular weight of monoclonal antibody was set to 150,000.

It was ensured that along with increasing of the added primary antibody, the binding reaction was saturated and the fluorescent intensity reached a constant amount. For calculating the number of the antigen on the cell surface and dissociation constant (Kd value), schatchard analysis (See, Scatchard, G.; Ann.N.Y.Acad.Sci.,51: 660-672,1949, New Cultured Cell Experiment Method for Molecular Biologic Research; Yodosha Co., Ltd., or Jikken-Igaku separate volume Bio manual UP series revision second version, 212-217). In this case, as the value, the average of the three points was used for each of the sample.

The binding between test antibody: Ligand (L) and Cell surface antigen: Receptor (R) are shown by the following reaction formula with Ligand Receptor complex (LR).

$$L + R \rightleftarrows LR$$

When Free Ligand concentration is [L] and non-binding type receptor concentration is [R] and Concentration of Ligand binding to Receptor is Kd, the dissociation constant Kd under equilibrium is as follows.

$$Kd = [L][R]/\ [LR] \quad \cdots\cdot I$$

The entire amount of used Ligand [L total] is the sum of the free type and the bound type.

$$[L\ total] = [L] + [LR] \quad \cdots\cdot II$$

By contrast, also, the total amount of Receptor [R total] is the sum of the receptor not binding to Ligand and the receptor binding to Ligand.

$$[R\ total] = [R] + [LR] \quad \cdots\cdot III$$

The number of the Receptor [R total] (maximum binding amount of the test antibody) and the affinity between Receptor and Ligand are estimated by Scatchard analysis. Then, the formulas of I, II, III were modified and substituted.

$$[LR]/[L] = -1/Kd[LR] + [R\ total]/Kd$$

where [LR] is represented as B (bound), [L] is represented as F (free), and [R total] is represented as Bmax (constant value).

$$B/F = -1/KdB + Bmax/Kd$$

The formula can be obtained. This formula is a linear function when B/F is set to y axis and B is set to x axis. From the inclination of the straight line, Kd value can be obtained, and from the y-intercept, Bmax can be obtained. The Kd value has the dimension of concentration, and the lower value thereof indicates higher affinity. (binding constant Ka = 1/Kd)

<Results>

When the affinity measurement was performed by using positive control (2B8) and test antibody A (antibody produced by Clone 1k1773 of 1K17 series of Table 1) and test antibody B (antibody produced by 1k1782 of 1K17 series, similarly), the image by the image analyzer is shown in Fig. 2 and the saturation curve is shown in Fig. 3 and the result of Scatchard analysis is shown in Fig. 4. Moreover, dissociation constant (Kd value) was obtained from the slope of the linear function obtained by the above Scatchard analysis. By repeating the same experiments, the obtained Kd values (averages) were as follows.

Table 2

| Antibody to be tested | Positive control 2B8 | Test antibody A 1k1773 | Test antibody B 1k1782 |
|---|---|---|---|
| Kd value (nM) | 6.8 | 1.3 | 0.40 |

Kd value obtained with respect to the antibody 2B8 used as the positive control was 6.8 nM. Because Kd value of 2B8 reported in Mitchell ER et al., Blood 1994; 82:435-445 is 3.5 nM, the approximate Kd value can also be obtained in the affinity measurement method of the present invention.

## Claims

1. A method for measuring binding affinity between an antibody to be measured and a cell membrane surface antigen, which comprises (a) binding the antibody to be measured to a floating cell presenting the antigen; (b) separating the antibody to be measured bound to the cell membrane surface antigen of the floating cell from the free antibody to be measured; (c) binding a labeled substance that binds to the antibody to be measured at a site different from the antigen recognizing site thereof, to the antibody to be measured bound to the cell membrane surface antigen of the floating cell; (d) separating the substance binding to the antibody to be measured bound to the cell membrane surface antigen of the floating cell from the free substance; and (e) detecting the label of the substance; and wherein the separations of (b) and (d) are each performed by centrifugation or through a filter through which a cell cannot pass.

2. The method according to claim 1, wherein the separations of (b) and (d) are performed by centrifugation.

3. The method according to claim 1, wherein the separations of (b) and (d) are performed through the filter through which a cell cannot pass.

4. The method according to any one of claims 1 to 3, wherein the floating cell is a normal animal cell, an animal cell strain made to be a cell line, or an animal cell strain modified genetically.

5. A method for assaying a monoclonal antibody that binds to a cell membrane antigen by utilizing the method according to any one of claims 1 to 4.

6. A method for screening a monoclonal antibody that binds to a cell membrane antigen by utilizing the method according to any one of claims 1 to 4.

FIG. 1

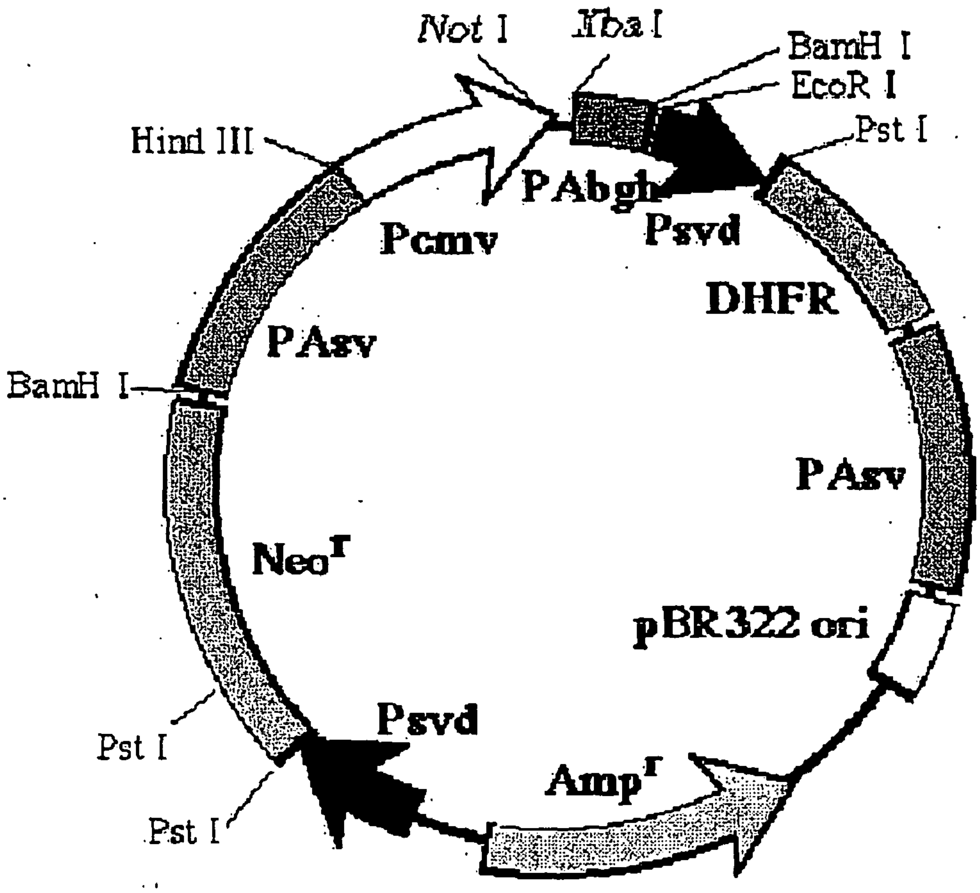


pNOW

| | |
|---|---|
| Pcmv: | CMV Promotor |
| Pabgh: | PolyA Addition Signal of Growth Hormone Gene |
| Psvd: | Modified SV40 Promoter |
| DHFR: | Mouse Dihydrofolate Reductase cDNA |
| Pasv: | Poly-A Addition Signal of SV40 Gene |
| PBR322ori: | Replication Origin in *Escherichia coli* |
| Ampr: | Selective Marker in *Escherichia coli* (Ampicillin Resistance) |
| Neor: | Selective Marker in Mammalian Cell (G418 Resistance) |

FIG. 2

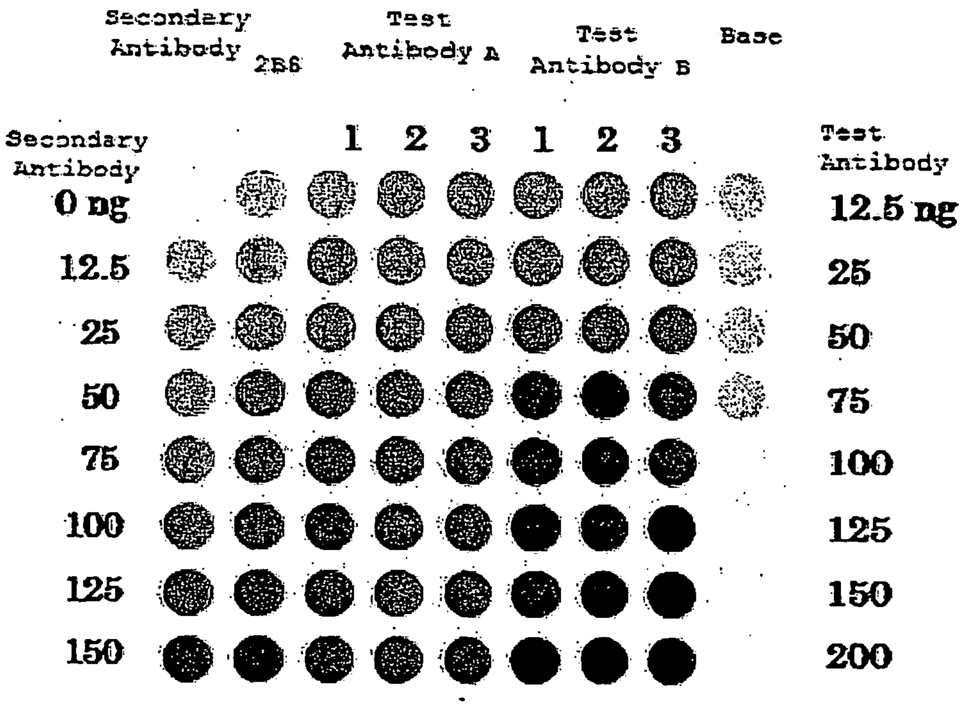
Secondary Antibody
2B6
Test Antibody A
Test Antibody B
Base
1 2 3 1 2 3
Secondary Antibody
0 ng
12.5
25
50
75
100
125
150
Test Antibody
12.5 ng
25
50
75
100
125
150
200

FIG. 3

Test Antibody A (1k1773)

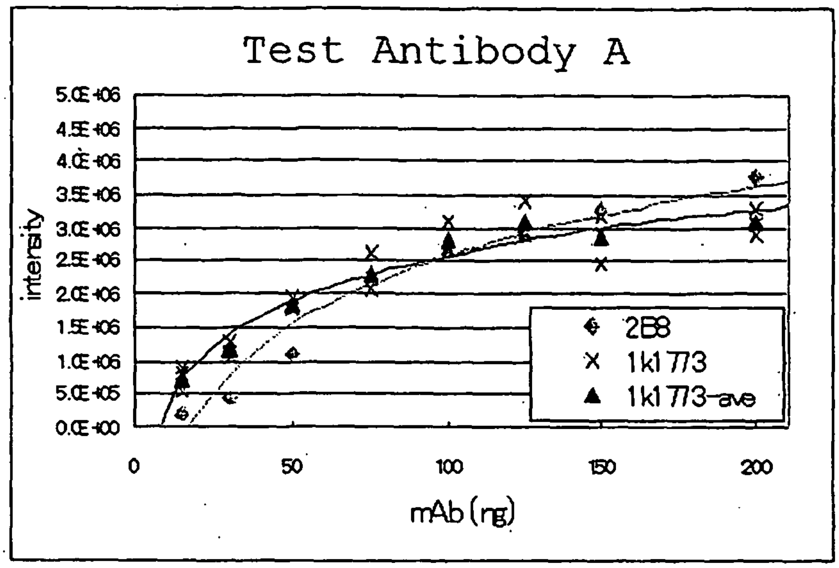


Test Antibody B (1k1782)

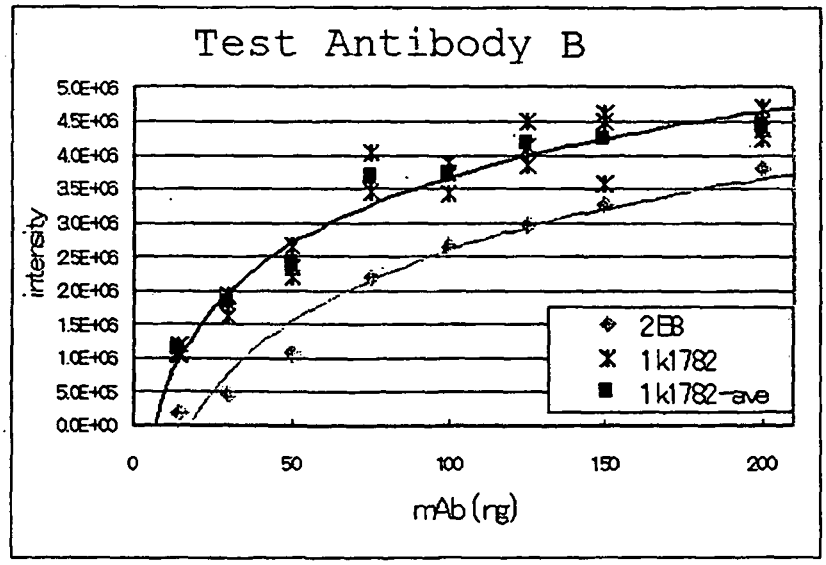

FIG. 4

Test Antibody A (1k1773)

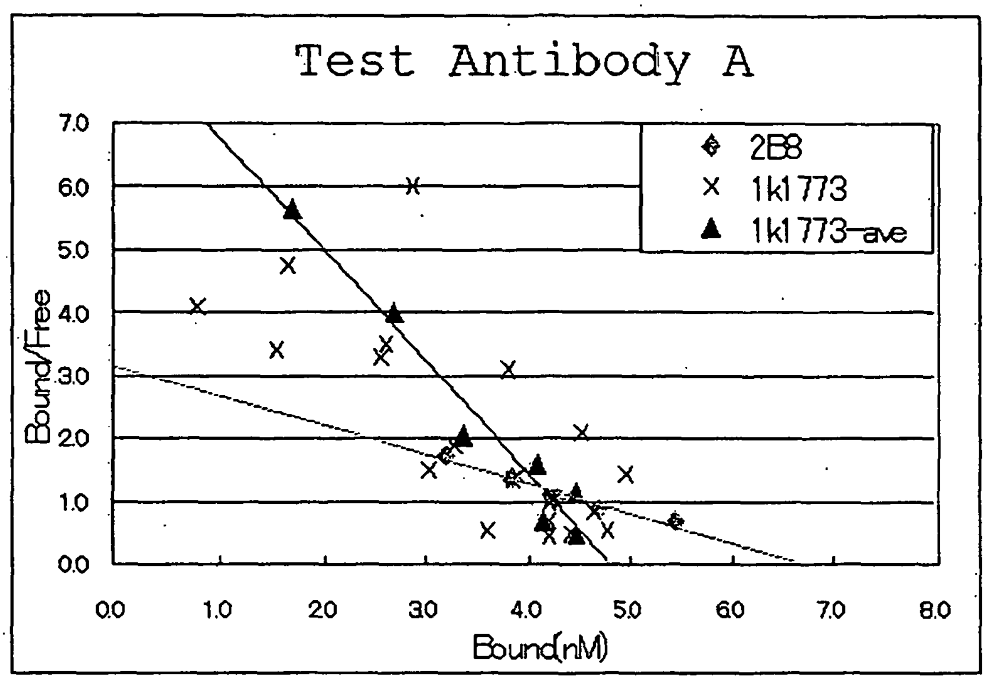


Test Antibody B (1k1782)

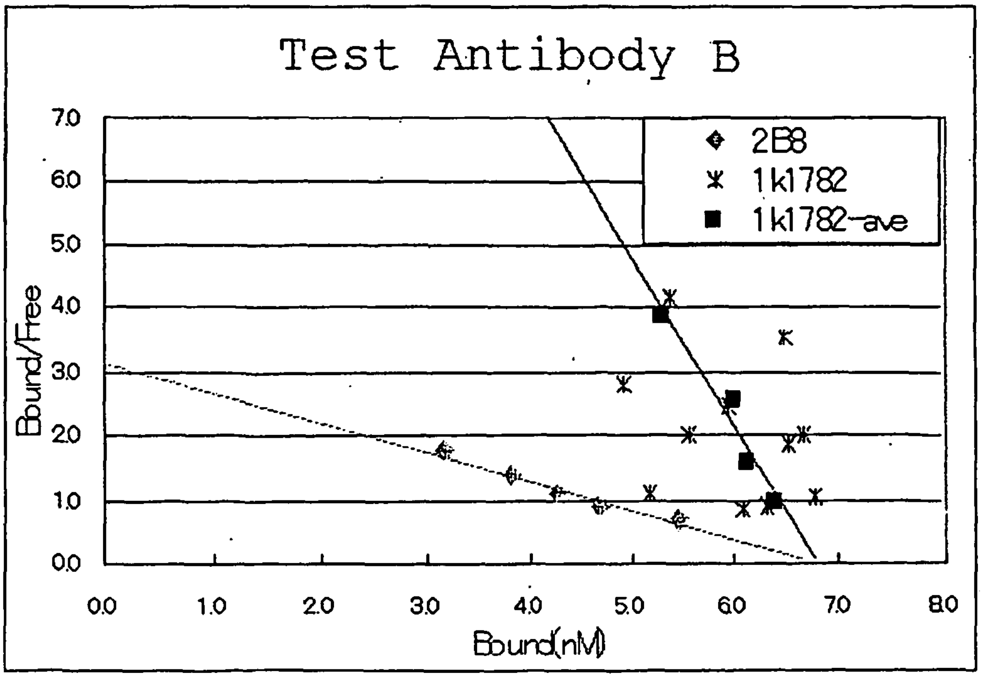

Europäisches Patentamt
European Patent Office
Office européen des brevets

# EUROPEAN SEARCH REPORT

Application Number
EP 09 00 7120

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BATOR J M ET AL: "Measurement of antibody affinity for cell surface antigens using an enzyme-linked immunosorbent assay" JOURNAL OF IMMUNOLOGICAL METHODS, ELSEVIER SCIENCE PUBLISHERS B.V.,AMSTERDAM, NL, vol. 125, no. 1-2, 20 December 1989 (1989-12-20), pages 167-176, XP023973832 ISSN: 0022-1759 [retrieved on 1989-12-20] * the whole document * ----- | 1-6 | INV. C12N15/02 C12N5/06 C12P21/08 G01N33/566 |
| A | CUNNINGHAM R E: "Indirect immunofluorescent labeling of viable cells" METHODS IN MOLECULAR BIOLOGY, HUMANA PRESS INC, NJ, US, vol. 115, 1 January 1999 (1999-01-01), pages 261-263, XP009120052 ISSN: 1064-3745 * the whole document * ----- | 1-6 | |
| | | | TECHNICAL FIELDS SEARCHED (IPC) |
| A | WILLINGHAM M C: "Fluorescence labeling of surface antigens of attached or suspended tissue-culture cells" METHODS IN MOLECULAR BIOLOGY, HUMANA PRESS INC, NJ, US, vol. 34, 1 January 1994 (1994-01-01), pages 123-130, XP009119963 ISSN: 1064-3745 * the whole document * ----- | 1-6 | C07K |
| A | JAVOIS L C: "Direct immunofluorescent labeling of cells" METHODS IN MOLECULAR BIOLOGY, HUMANA PRESS INC, NJ, US, vol. 115, 1 January 1999 (1999-01-01), pages 107-111, XP009120064 ISSN: 1064-3745 * page 107 - page 108; figure 1 * ----- | 1-6 | |

The present search report has been drawn up for all claims

| Place of search | Date of completion of the search | Examiner |
|---|---|---|
| Munich | 17 July 2009 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Protein Expression Technologies: Current Status and Future Trends. BIOS Scientific Publishers, 01 April 2004
- Membrane Protein Protocols: Expression, Purification, and Characterization. Methods in Molecular Biology. 01 June 2003, vol. 228
- **Julie P.D. et al.** *Imminol,* 2002, vol. 107, 176-82
- **Maria J. Polyak ; Julie P. Deans.** *Studies of Existing CD Molecules,* 93-95
- **M. S. Cragg et al.** *Studies of Existing CD Molecules,* 95-97
- **Anderson KC et al.** Blood. 1984, vol. 63, 1424-33
- **Oi,V.T. ; L.A. Herzenberg.** Selected Methods in Cellular Immunology. Freeman and Co, 1980, 351
- **Mitchell ER et al.** *Blood,* 1994, vol. 82, 435-445